# EUROPEAN PATENT APPLICATION

(11) **EP 3 669 882 A2**
(43) Date of publication of application: **24.06.2020**
(21) Application number: 20153682.8
(22) Date of filing: 28.02.2012
(51) Int. Cl.: A61K 31/732, A61K 31/734, A61P 37/04, A61P 35/02

(54) **ACTIVATION OF HUMAN T-HELPER/INDUCER CELL, T-CYTOTOXIC/SUPPRESSOR CELL, B-CELL, AND NATURAL KILLER (NK)-CELLS AND INDUCTION OF NATURAL KILLER CELL ACTIVITY AGAINST K562 CHRONIC MYELOID LEUKEMIA CELLS WITH MODIFIED CITRUS PECTIN**

(30) Priority: 28.02.2011 US 201161447138 P
(62) Divisional of application: 12789021.8
(71) Applicant: Econugenics, Inc., Santa Rosa, CA 95401 (US)
(72) Inventor: ELIAZ, Isaac, Sebastopol, CA California 95472 (US)
(74) Representative: J A Kemp LLP

(57) **Abstract**

Modified Citrus Pectin is comprised of short, slightly -branched carbohydrate chains derived from citrus fruit peels alter by decreasing the molecular weight and degree of esterification. MCP is demonstrated herein to induce the activation of T-cytotoxic/suppressor cells, B-cells and NK-cells. Administration of MCP to mammals offers a method to enhance immune system responses of this kind in mammals in need of the same using safe and familiar materials.

## Description

### Priority Data and Incorporation by Reference

This application claims benefit of priority to U.S. Provisional Patent Application No. 61/447,138 filed February 28, 2011 which is incorporated by reference in its entirety.

### BACKGROUND OF THE INVENTION

### Field of the Invention

The present invention relates to Modified citrus pectin (MCP). MCP is known for its anti-cancer effects and its ability to be absorbed and circulate in the human body. We here demonstrate the ability of this MCP to induce the activation of human blood lymphocyte subsets including T-helper/inducer, T-cytotoxic/suppressor cell, B-cell, and Natural Killer (NK)-cells, and the induction of NK-cell activity against K562 chronic myeloid leukemia cells. A carbohydrate composition analysis is performed to propose a structural mechanism of action of immune enhancement by MCP.

Pectins, and related alginates, have been demonstrated to be safe and effective and have powerful influence in treating various conditions in mammals. U.S. Patents 6,274,566, 6,462,029, 7,026,302 and 7,452,871, all by the named inventor herein, demonstrate the ability of low molecular weight modified pectin to bind to a variety of different agents in the circulation, including tumor emboli, toxins, heavy metals and calcium, thereby reducing the spread of metastatic cancer and slowing atherosclerosis, and ameliorating conditions caused by these agents. These four patents are incorporated herein-by-reference

U.S. Patent Publication U.S. 20070167395 provides data showing the administration of MCP in test animals to be effective in treatment of diabetes. U.S. Patent Publication U.S. 20070049551 demonstrates the effectiveness of MCP as an agent to induce a generalized immune response in mammals lacking an adequate immune response. U.S. Patent Publication U.S. 20110294755 describes the effectiveness of MCP in binding Galectin-3, to address inflammation and the myriad of conditions associated with inflammation. The disclosures of all three of these patent publications are incorporated-herein-by-reference.

### Background of the Invention

### Methods

Human blood samples were collected and incubated with increasing concentrations of MCP along with appropriate positive controls. The blood samples were mixed with antibody, incubated, lysed and run on a Coulter Elite flow cytometer using a 3-color protocol. The percentage of activated T-helper/inducer cells, T-cytotoxic/suppressor cells, B-cells and NK-cells and the percentage increase over untreated control were calculated and plotted against MCP concentrations.

To test the functionality of the activated NK-cells, normal lymphocytes were isolated, washed, plated and treated with increasing concentrations (0-800 ug/ml) of MCP and positive controls. Log-phase PKH26-labeled K562 leukemic cells (0.2 x 10⁶ cells) were added to the normal lymphocytes and incubated for another 4 hours for inducing cell death. The cell mixture was permeabilized, washed, and stained with a human specific FITC-labeled active form of caspase 3 antibody. The stained cells were washed, and analyzed by a two color flow cytometry protocol with FL1 and FL2 measuring PKH26 and FITC, respectively. The percentage of K562 cells positive for PKH26 and FITC were calculated as the dead cells induced by NK-cells.

Statistical analysis: Mean and standard deviation values were calculated and graphs prepared by Microsoft Excel. Kruskal-Wallis one-way Analysis of Variance (ANOVA) was used to analyze the data and *p* values were estimated by the GraphPad Prism software.

Monosaccharide analysis of the MCP was performed by high-performance anion-exchange chromatography with pulse amperometric detection (HPAEC-PAD). Weight average molar mass and intrinsic viscosity analysis was performed by high-performance size-exclusion chromatography (HPSEC) with multiple detectors (multi-angle laser light scattering, refractive index and differential pressure viscometer).

### Results

Results show that MCP does not have a significant effect on T-helper/inducer cell activation as compared to positive control. However, results show that MCP activated T-cytotoxic/suppressor cells at low levels and in a dose-dependent manner, B-cell in a significant dose-dependent manner, and induced significant dose-dependent activation of NK-cells. MCP-Activated NK-cells demonstrated functional activity in inducing cancer cell death. The MCP consisted of oligogalacturonic acids with some containing 4,5-unsaturated non-reducing ends. Unsaturated oligogalacturonic acids have been reported with immunomodulatory activity.

### Conclusions

The data demonstrates that MCP is a substance with strong immunostimulatory properties in human blood samples, including the activation of functional NK cells against K562 leukemic cells in culture. Unsaturated oligogalacturonic acids appear to be the immunostimulatory carbohydrates in MCP. Additional *in vivo* studies to better understand the applications of MCP as an immune enhancer are warranted.

### SUMMARY OF THE INVENTION

Pectin is a complex carbohydrate soluble fiber. Dietary fibers, such as pectin, have been shown to have positive effects on a wide spectrum of pathological conditions. Their positive influence on human health is explained by their anti-oxidative, hypocholesterolemic, and anti-cancer effects [1-12]. The effect on the immune system has been previously attributed to the down regulation of the inflammatory response, moderating the production of pro inflammatory cytokines and immunoglobulins in murine models for irritable bowel syndrome [13]. A diet rich in soluble fiber in an animal model showed protection against endotoxin-induced sickness behavior by cytokine modulation and promotion of alternative activation of macrophages [14]. Citrus pectin has the capacity to exert a favourable immunomodulatory response in human peripheral blood cells through its effect on cytokine production [15]. High methoxy citrus pectin inhibits the binding of fibroblast growth factor-1 (FGF-1) to its receptor in the presence of heparin [16]. The rhamnogalacturonan I-arabinan fraction of pectin from a medicinal herb enhances secretion of granulocyte colony-stimulating factor (G-CSF) by murine colonic MCE 301 cells [17]. Rhamnogalacturonan I-arabinogalactan was also reported to activate macrophages and dendritic cells [18]. Methyl-esterified pectic oligosaccharides with 4,5-unsaturated non-reducing ends enhanced T-helperl (Th1) dependent delayed-type hypersensitivity in a murine influenza vaccine model, reduced Th2 cytokine (IL-4, IL-5 and IL-10) production in splenocytes *in vitro* [19] and decreased allergic asthma in mice [20]. Therefore, the carbohydrate composition of pectin is very important in determining different immune responses.

The modified citrus pectin (MCP) used in this study, is composed of short, slightly-branched, carbohydrate chains derived from the soluble albedo fraction of citrus fruit peels altered by decreasing the molecular weight and degree of esterification using pH, temperature and a controlled enzymatic process, in order to increase its absorption into the circulatory system. MCP is relatively rich in galactose, and antagonizes a binding protein galectin-3 (Gal-3), which results in suppression of cancer cell aggregation, adhesion, and metastasis [5-6]. MCP acts as a ligand for galectin-3, which plays a major role in tumor formation and progression [12, 21-24]. It has been shown using a combination of fluorescence microscopy, flow cytometry, and atomic force microscopy, that specific binding of a pectin galactan to the recombinant form of human galectin-3 has been physically observed [25]. Moreover, MCP also showed anti-metastatic effects on cancer cells *in vitro* or *in vivo* [8, 10-11,24,26-28]. Human clinical trials with MCP showed an increase in prostate specific antigen doubling time, a marker of slowing the progression of prostate cancer [9], and significant improvement of quality of life and stabilization of disease for patients with advanced solid tumors [29]. Besides the therapeutic roles against cancer, MCP has been shown to also remove toxic metals from the body [30-31]. In the United States of America, MCP is registered as a food supplement and is generally regarded as safe (GRAS).

*In vitro* lymphocyte activation represents a standard approach for evaluating cell-mediated responses to a variety of stimuli including immunostimulatory botanical extracts. An appropriate assay system monitors the expression of the early activation marker CD69 in whole blood after stimulation with extracts. CD69 is expressed in all activated lymphocyte subsets and hence it represents a generic marker to monitor individual subset responses to specific stimuli.

T-lymphocyte subsets can be identified and quantified by using fluorochrome-labeled antibody combinations such as CD4/CD69/CD3 and CD8/CD69/CD3. CD4 antigen is expressed on the T- helper/inducer lymphocyte subset (CD3/CD4). CD8 antigen is expressed on the human suppressor/cytotoxic T-lymphocyte subset (CD3/CD8). Once activated both CD4 and CD8 positive T cells express CD69.

CD19 antigen is present on human B-lymphocytes at all stages of maturation and is not present on resting or activated T-lymphocytes. CD19/CD69/CD45 combination can be used to identify an activated B cell population. CD56 antigen is present on Natural Killer (NK)-cells and antigen intensity increases with NK-cell activation. Hence, CD56/CD69/CD45 combination can be used to identify activated NK-cells.

The natural killing ability of NK-cells is considered an indicator of the immune stimulating ability of supplements and natural products. Therefore, the ability of NK-cell subset in normal lymphocytes to induce death in leukemia cells is analyzed by co-incubating MCP treated lymphocytes with K562 T-cell leukemia cells.

In this report we tested the ability of MCP to induce the activation of human blood lymphocyte subsets (T-helper/inducer, T-cytotoxic/suppressor, B-cell, and NK-cell), and the induced NK-cell's activity against K562 chronic myeloid leukemia cells. Carbohydrate composition analysis was performed to propose a structural mechanism of action of this immune enhancement.

### BRIEF DESCRIPTION OF THE DRAWINGS

The accompanying drawings, which are incorporated herein and constitute part of this specification, illustrate exemplary embodiments of the invention, and, together with the general description given above and the detailed description given below, serve to explain the features of the invention.
FIG. 1 is - Figure 1 - Showing the Effect of MCP on T-Helper/Inducer Cell Activation in Blood Cultures *p* < 0.05 analyzed by ANOVA and compared by critical difference value.
FIG 2 is - Figure 2 - Demonstrating the iIncrease in T-Helper/Inducer Cell Activation (%) by MCP
   *p* < 0.05 analyzed by ANOVA and compared by critical difference value.
FIG 3 is - Figure 3 - Illustrating the eEffect of MCP on T-Cytotoxic/Suppressor Cell Activation in Blood Cultures: *p* < 0.05 analyzed by ANOVA and compared by critical difference value.
FIG 4 is - Figure 4 - Reflecting the increase in T-Cytotoxic/Suppressor Cell Activation (%) by MCP *p* < 0.05 analyzed by ANOVA and compared by critical difference value.
FIG 5 is - Figure 5 - Demonstrating the Effect of MCP on B-Cell Activation in Blood Cultures
   *p* < 0.05 analyzed by ANOVA and compared by critical difference value.
FIG 6 is - Figure 6 - Setting forth the increase in B-Cell Activation (%) by MCP *p* < 0.05 analyzed by ANOVA and compared by critical difference value.
FIG 7 is - Figure 7 - A table showing the Effect of MCP on NK-Cell Activation in Blood Cultures
   *p* < 0.05 analyzed by ANOVA and compared by critical difference value.
FIG 8 is - Figure 8 - Demonstrating an increase in NK-Cell Activation (%) by MCP *p* < 0.05 analyzed by ANOVA and compared by critical difference value.
FIG 9 is - Figure 9 - Induction of NK-Cell Activity by MCP *p* = 0.0082 by Kruskal-Wallis non-parametric ANOVA.
FIG 10 is - Figure 10 - Increase in Percent NK-Cell Activity by MCP *p* = 0.0082 by Kruskal-Wallis non-parametric ANOVA.
FIG 11 is - Figure 11 - HPAEC-PAD analysis of MCP (1) compared to a polygalacturonic acid hydrolysate (2). The degree of polymerization is listed over the peaks. The arrows point to unsaturated oligogalacturonic acids

### DETAILED DESCRIPTION OF THE INVENTION

MCP (PectaSol-C® MCP, Econugenics, Inc., Santa Rosa, CA, USA) was initially solubilized in phosphate buffered saline (PBS). The solubility of MCP in PBS was 76.4%. The volume was adjusted to get accurate amounts of each compound for treatment based on solubility factor.

T-, B- and NK-cell activation assay: Blood samples were collected from healthy volunteers and 250 µl blood samples were incubated in 48-well plates with increasing concentrations of compound along with appropriate positive controls (recommended by Becton Dickinson Biosciences, CA, USA) for each subset. CD2/CD2R and Phorbol ester (PMA) were used as controls for T-cytotoxic/suppressor cell activation studies. Pokeweed (PWM) mitogen was used as positive control for B-cell activation and IL-2 was used for activating NK-cells in blood cultures. The blood cultures were incubated at 37°C in a CO₂ incubator for 24 h. On the next day, 20 µl of specific antibody mix, [CD4-FITC/CD69-PE/CD45-PerCP (T-helper/inducer cell activation), CD8-FITC/CD69-PE/CD3-PerCP (T-cytotoxic/suppressor cell activation), CD19-FITC/CD69-PE/CD45-PerCP (B-cell activation) and CD56-FITC/CD69-PE/CD45-PerCP (NK-cell activation)] was dispensed into separate flow tubes and 50 µl blood sample was mixed with antibody and incubated for 30 min at room temperature in the dark. The blood-antibody mix was lyzed in a Coulter Epics Q-prep work station using Immunoprep kit and run on a Coulter Elite flow cytometer using a 3-color protocol. The percentage of activated T- cytotoxic/suppressor cells, B-cells and NK-cells and the percentage increase over untreated control were calculated and plotted against compound concentrations.

Functional NK Cell Activity Assay: Normal lymphocytes were isolated from three healthy volunteers using histopaque 1077 gradient centrifugation. The cells were washed twice with PBS and re-suspended in phenol-free RPMI 1640 medium supplemented with 10% fetal bovine serum and antibiotics (complete medium). Cells were plated in special 48-well deep plates (conical bottom) at 10⁶ cells/ml/well and treated with increasing concentrations (0-800 ug/ml) of MCP in a 5% CO₂ incubator at 37°C for 24 h. On the next day, log-phase K562 leukemic cells were labeled with PKH26 membrane dye (Sigma, St. Louis, MO, USA) for 5 min according to manufacturer's protocol and 0.2 x 10⁶ cells each were added to the normal lymphocytes in the 48 well plates. The plates were centrifuged for 1 min at 250 xg and returned to the CO₂ incubator for another 4 hours for inducing cell death.

The cell mixture was permeabilized by incubating in 2% paraformaldehyde solution at 4°C followed by incubation in 0.2% Tween 20 solution (PBS) at 37°C. The cell mixture was washed with ice-cold PBS and stained with human specific FITC-labeled active form of caspase 3 antibody for 30 minutes at room temperature according to the procedure published earlier [32-34]. The stained cells were washed with 0.1% Tween 20 solution, resuspended in 0.5 ml of staining buffer and analyzed by a two-color flow cytometry protocol with FL1 and FL2 measuring PKH26 and FITC, respectively in a Beckman Coulter Elite Flow cytometer. The percentage of K562 cells positive for PKH26 and FITC are dead cells induced by NK-cells.

Statistical Analysis: Mean and standard deviation values were calculated and graphs prepared by Microsoft Excel. Kruskal-Wallis one-way Analysis of Variance (ANOVA) was used to analyze the data by the GraphPad Prism software and *p* values were estimated.

Carbohydrate Composition Analysis of MCP: Monosaccharide analysis was performed by high-performance anion-exchange chromatography with pulse amperometric detection (HPAEC-PAD) following methanolysis according previously published method [31]. HPAEC-PAD was also used for oligosaccharide analysis [31]. Weight average intrinsic viscosity analysis was performed by high-performance size-exclusion chromatography (HPSEC) with multiple detectors (multi-angle laser light scattering, refractive index and differential pressure viscometer according to previously publish methods [30].

### Results and Discussion

### T-Lymphocyte Subset Activation

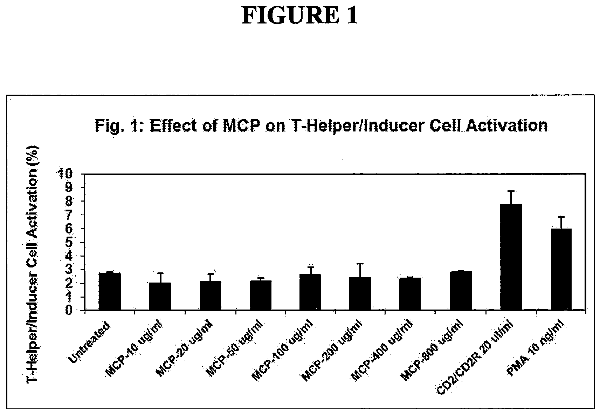

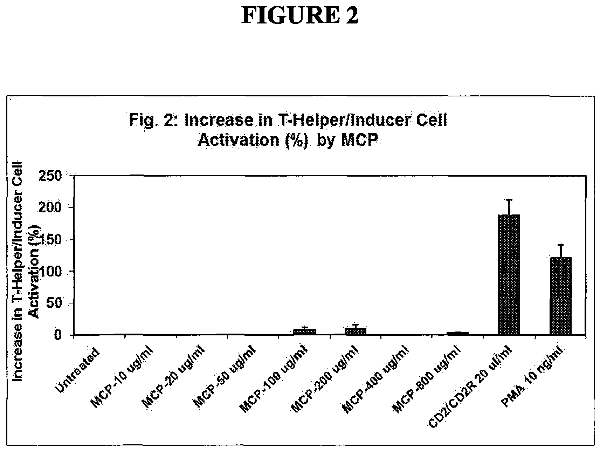

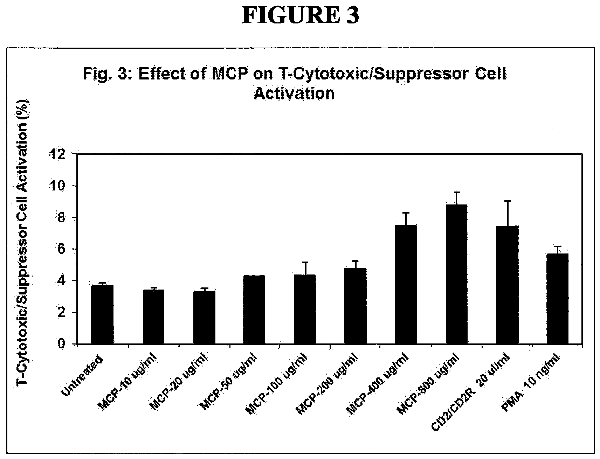

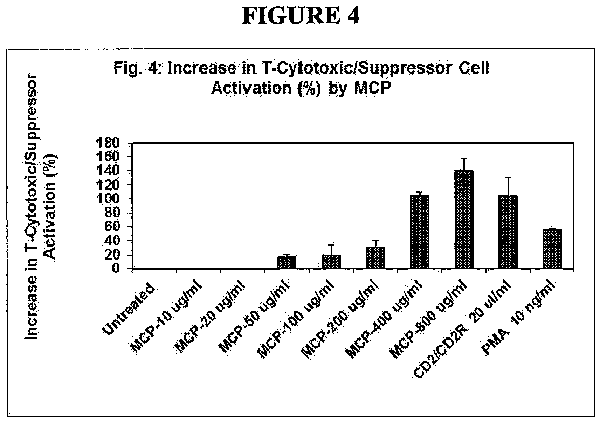

The effects of compound on T-helper/inducer and T-cytotoxic/suppressor cell activation are shown in Fig. 1 & 3, respectively. The percent increases in T- helper/inducer lymphocyte and T-cytotoxic/suppressor cell activation are shown in Fig. 2 & 4, respectively. Results show that MCP does not have a significant effect on T-helper/inducer cell activation as compared to positive controls like CD2/CD2R and PMA. However, MCP activated T-cytotoxic/suppressor cells at lower levels and in a dose-dependent manner between 50-800 ug/ml concentrations. In this case, all positive controls induced expected responses.

### B-Cell Activation

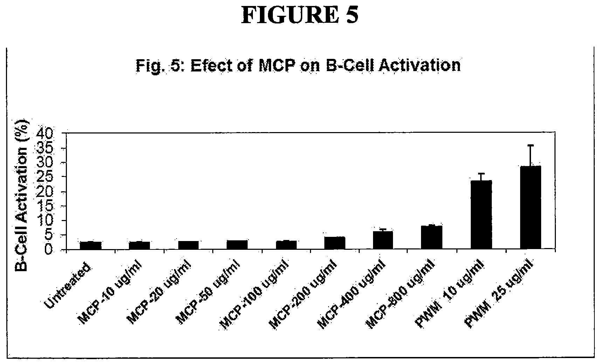

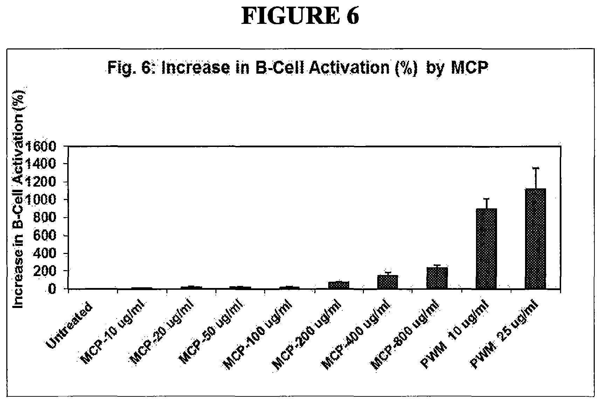

The effects of MCP on B-cell activation and percentage increase over untreated control are given in Fig. 5 & 6, respectively. The level of B-cell activation by MCP although lower than the positive control PWM, a dose-dependent and significant increase was noticed.

### NK-Cell Activation

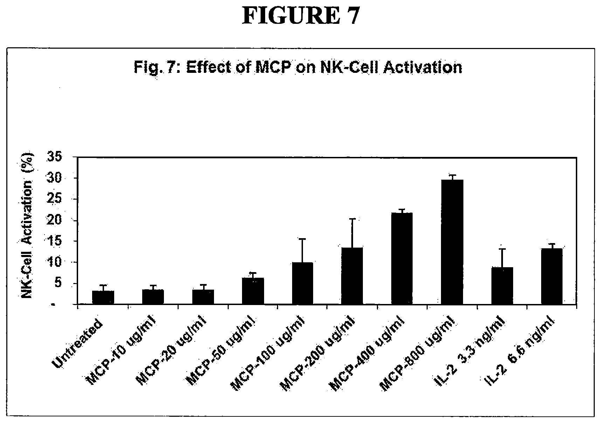

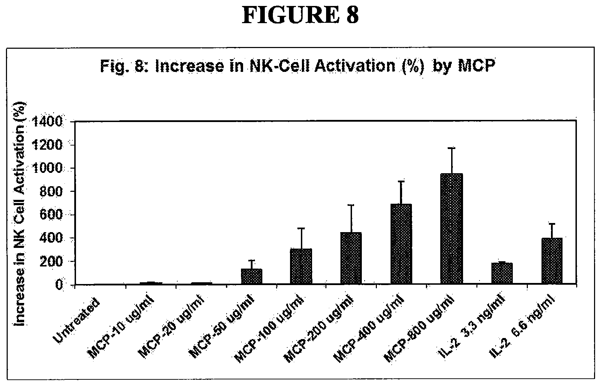

The effects of MCP on NK-cell activation data and the percentage increase over untreated control are given in Fig. 7 & 8, respectively. The positive control IL-2 induced expected results. MCP demonstrated a significant dose-dependent activation of NK-cells showing its immunostimulatory potential of the compound.

MCP activated NK-Cell activity on K562 Chronic Myeloid Leukemia Cells

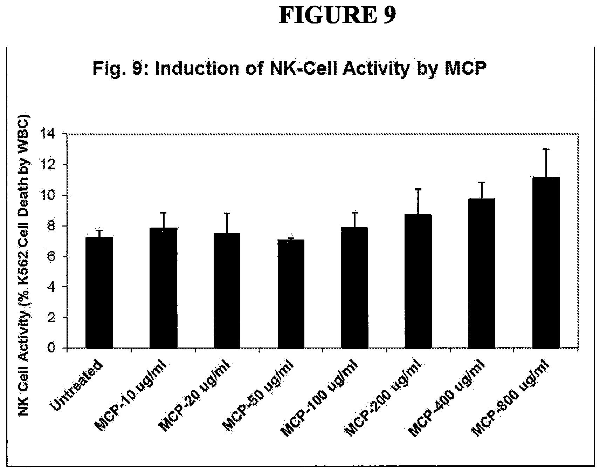

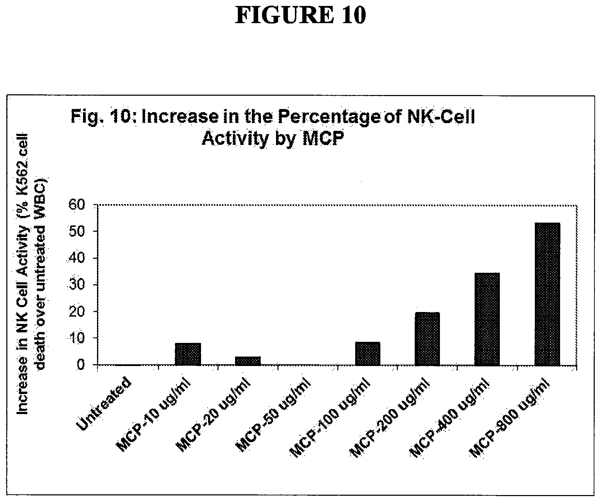

The results of MCP treated blood samples on induction and increase in percent of NK-cell activity assay are shown in Fig. 9 & 10, respectively. MCP induces NK-cell activity in a dose-dependent manner with 800 ug/ml dose causing about 53.60% increase in NK-cell activity. Increase in NK-cell activity corresponds with the activation of NK-cells obtained by staining with NK-cell activation markers (CD56/CD69/CD45).

### MCP Carbohydrate Composition Analysis

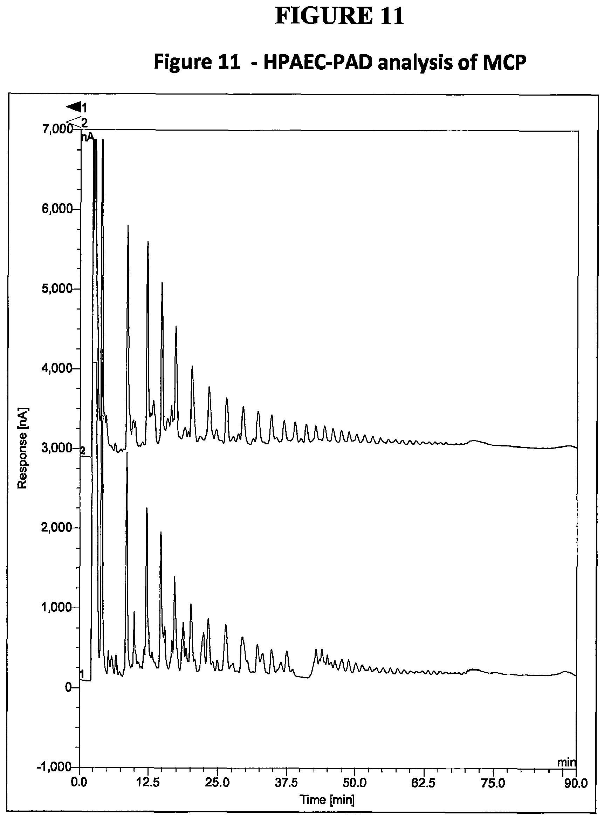

The monosaccharide composition of the MCP was identified and is reported in Table 1. The MCP consisted of mainly galacturonic acid with galactose and arabinose the most common neutral sugars. The MCP had a high amount of homogalacturonan compared to rhamnogalacturonan (high GalA/Rha ratio). On average the homogalacturonan backbone of the MCP was interrupted with a rhamnose residue once every 22 galacturonic acid residues (Table 1). Arabinan, galactan or arabinogalactan neutral sugar side-chains in pectin are attached to the rhamnose residues in rhamnogalacturonan. The homogalacturonan consisted of both saturated and unsaturated oligogalacturonic acids (Fig. 11). The unsaturated oligogalacturonic acids had longer retention times compared to saturated oligogalacturonic acids [32]. Unsaturated oligogalacturonic acids were not detected in MCP previously [30]. Except for lower degree of esterification, the unsaturated oligogalacturonic acids in MCP are the same pectic oligosaccharides as those reported to polarize the T-helper response toward Th1 in a murine influenza vaccination model [19]. MCP average molar mass, intrinsic viscosity, and Mark Houwink constant are reported in Table 2. The weight average intrinsic viscosity of pectin is directly proportional to the molar mass, thus demonstrating a lower molecular weight range of the MCP compared to 14,800 and 0.398 dL/g reported previously [30]. The lower Mark Houwink constant for MCP compared to the 1.45 that we observed previously [30] means that the global structure has more random coil shape typical of citrus pectin, while a higher Mark Houwink constant was interpreted as being more rigid and rod like. Therefore, MCP is a low molecular weight pectin enriched in saturated and unsaturated oligogalacturonic acids.

The immune system protects people from disease causing microorganisms and other harmful materials. Circulating blood transports immune components between organs of the immune system and sites of inflammation. The enumerative (activation marker assays) and functional assays (NK cell activity assay) used in the present study to evaluate the immunostimulative potential of MCP clearly demonstrated activation of T cytotoxic/suppressor, B and NK cells in blood cultures *in vitro.* Furthermore, functional assay indicated that MCP enhanced cytolysis of leukemic cells by NK cells. It is well known that NK cell activity is regulated by the expression levels of cytotoxic molecules, activating receptors and/or inhibitory receptors. Even though several CAM agents have been shown to activate NK-cells and improve NK cell activity, the precise mechanism is not clearly defined. The two main possibilities are: (i) augmentation of cytotoxic molecules in NK cells and/or (ii) up-regulation of activating NK receptors and/or down-regulation of inhibitory NK receptors [38,39].

### Conclusions

The data shows MCP as a substance with strong immunostimulatory properties in human blood samples, including the activation of functional NK cells against K562 leukemic cells in culture. The immune stimmulation properties are proposed to be attributed to the presence of low degree of methyl esterification unsaturated oligogalacturonic acids. Additional research may be of value in demonstrating that changing the oligogalacturonic acid degree of esterification alters the immune response in mammals.

### List of abbreviations

### MCP: Modified citrus pectin

NK: Natural killer
SD: Standard deviation
PBS: Phosphate buffered saline
DMSO: Dimethyl sulfoxide

### References

1. Anderson JW, Jones AE, Riddell-Mason S: Ten different dietary fibers have significantly different effects on serum and liver lipids of cholesterol-fed rats. J Nutr 1994, 124:78-83.
2. Anderson JW: Dietary fiber, complex carbohydrate and coronary artery disease. Can J Cardiol 1995, 11:55G-62G.
3. Bingham SA, Day NE, Luben, R, Ferrari P, Slimani N, Norat T, Clavel-Chapelon F, Kesse E, Nieters A, Boeing H, Tjønneland A, Overvad K, Martinez C, Dorronsoro M, Gonzalez CA, Key TJ, Trichopoulou A, Naska A, Vineis P, Tumino R, Krogh V, Bueno-de-Mesquita HB, Peeters PH, Berglund G, Hallmans G, Lund E, Skeie G, Kaaks R, Riboli E: Dietary fiber in food and protection against colorectal cancer in the European Prospective Investigation into Cancer and Nutrition (EPIC): an observational study. Lancet 2003, 361:1496-1501.
4. Chen CH, Sheu MT, Chen TF, Wang YC, Hou WC, Liu DZ, Chung TC, Liang YC: Suppression of endotoxin induced proinflammatory responses by citrus pectin through blocking LPS signalling pathways. Biochem Pharmacol 2006, 72:1001-1009.
5. Nangia-Makker P, Conklin J, Hogan V, Raz A: Carbohydrate-binding proteins in cancer, and their ligands as therapeutic agents. Trends Mol Med 2002, 8:187-192.
6. Kidd P: A new approach to metastasis cancer prevention: modified citrus pectin (MCP), a unique pectin that blocks cell surface lectins. Altern Med Rev 1996, 1:4-10.
7. Olano-Martin E, Rimbach GH, Gibson GR, Rastall RA: Pectin and pectic-oligosaccharides induce apoptosis in in vivo human colonic adenocarcinoma cells. Anticancer Res 2003, 23:341-346
8. Nangia-Makker P, HoganV, Honjo Y, Baccarini S, Tait L, Bresalier R, Raz A: Inhibition of human cancer cell growth and metastasis in nude mice by oral intake of modified citrus pectin. J Natl Cancer Ins. 2002, 94 (24):1854-1862.
9. Guess BW, Scholz MC, Strum SB, Lam RY, Johnson HJ, Jennrich RI: Modified citrus pectin (MCP) increases the prostate-specific antigen doubling time in men with prostate cancer: a phase II pilot study. Prostate Cancer Prostatic Dis 2003, 6:301-304.
10. Pienta KJ, Naik,H, Akhtar A, Yamazaki K, Replogle TS, Lehr J, Donat TL, Tait L, Hogan V, Raz A: Inhibition of spontaneous metastasis in a rat prostate cancer model by oral administration of modified citrus pectin. J Natl Cancer Inst 1995, 87(5):348-353.
11. Yan J, Katz A: PectaSol-C modified citrus pectin induces apoptosis and inhibition of proliferation in human and mouse androgen-dependent and- independent prostate cancer cells. Integr Cancer Ther: 2010, 9(2):197-203.
12. Glinsky VV, Raz A: Modified citrus pectin anti-metastatic properties: one bullet multiple targets. Carbohydr Res 2009, 14:1788-91.
13. Ye MB, Lim BO: Dietary pectin regulates the levels of inflammatory cytokines and immunoglobulins in interleukin-10 knockout mice. J Agric Food Chem 2010, Oct 14. [Epub ahead of print]
14. Sherry CL, Kim SS, Dilger RN, Bauer LL, Moon ML, Tapping RI, Fahey GC Jr, Tappenden KA, Freund GG: Sickness behavior induced by endotoxin can be mitigated by the dietary soluble fiber, pectin, through up-regulation of IL-4 and Th2 polarization. Brain Behav Immun 2010,24(4):631-40.
15. Salman H, Bergman M, Djaldetti M, Orlin J, Bessler H: Citrus pectin affects cytokine production by human peripheral blood mononuclear cells. Biomed Pharmacother 2008, 62(9):579-82.
16. Liu Y, Ahmad H, Luo Y, Gardiner DT, Gunasekera RS, McKeehan WL, Patil BS. Citrus pectin: characterization and inhibitory effect on fibroblast growth factor-receptor interaction. J Ag Food Chem 2001, 49: 3051-3057.
17. Matsumoto T, Moriya M, Sakurai MH, Kiyohara H, Tabuchi Y, Yamada H: Stimulatory effect of a pectic polysaccharide from a medicinal herb, the roots of Bupleurm falcatum L., on G-CSF secretion from intestinal epithelial cells. Int Immunopharmacol 2008, 8:581-588.
18. Inngjerdingen M, Inngjerdingen KT, Patel TR, Allen S, Chen X, Rolstad B, Morris GA, Harding SE, Michaelsen TE, Diallo D, Paulsen BS: Pectic polysaccharides from Biophytum petersianum Klotzsch, and their activation of macrophages and dendritic cells. Glycobiology 2008, 18(12):1074-84.
19. Vos AP, Haarman M, van Ginkel JW, Knol J, Garssen J, Stahl B, Boehm G, M'Rabet L: Dietary supplementation of neutral and acidic oligosaccharides enhances Th1-dependent vaccination responses in mice. Pediatr Allergy Immunol 2007a; 18:304-312.
20. Vos AP, van Esch BC, Stahl B, M'Rabet L, Folkerts G, Nijkamp FP, Garssen J: Dietary supplementation with specific oligosaccharide mixtures decreases parameters of allergic asthma in mice. Int Immunopharmacol 2007b, 7:1582-1587.
21. Zou J, Glinsky VV, Landon L.A, Matthews L, Deutscher SL: Peptides specific to the galectin-3 carbohydrate recognition domain inhibit metastasis-associated cancer cell adhesion. Carcinogenesis 2005, 26:309-318.
22. Johnson KD, Glinskii OV, Mossine VV, Turk JR, Mawhinney TP, Anthony DC, Henry CJ, Huxley VH, Glinsky GV, Pienta KJ, Raz A, Glinsky VV: Galectin-3 as a potential therapeutic target in tumors arising from malignant endothelia. Neoplasia 2007, 9:662-670.
23. Nangia-Makker P, Honjo Y, Sarvis R, Akahani S, Hogan V, Pienta KJ, Raz A: Galectin-3 induces endothelial cell morphogenesis and angiogensis. Am J Pathol 2000, 156:899-909.
24. Inohara H, Raz A: Effects of natural complex carbohydrate (citrus pectin) on murine melanoma cell properties related to galectin-3 functions. Glycoconj J 1994, 11:527-532.
25. Gunning AP, Bongaerts RJ, Morris VJ: Recognition of galactan components of pectin by galectin-3. FASEB J 2009, 23:415-424.
26. Platt D, Raz,A: Modulation of the lung colonization of B16-F1 melanoma cells by citrus pectin. J Natl Cancer Inst 1992, 84:438-442.
27. Glinsky VV, Huflejt M, Glinsky G, Deutscher S, Quinn T: Effects of Thomsen-Friendenreich antigen-specific peptide p-30 on β-galactoside-mediated homotypic aggregation and adhesion to the endothelium of MDA-MB-435 human breast carcinoma cells. Cancer Res 2000, 60:2584-2588.
28. Liu HY, Huang ZL, Yang G.H, Lu WQ, Yu NR: Inhibitory effect of modified citrus pectin on liver metastases in a mouse colon cancer model. World J Gastroenterol 2008, 14:7386-7391.
29. Azemar M, Hildenbrand B, Haering B, Heim ME, Unger C: Clinical benefit in patients with advanced solid tumors treated with modified citrus pectin: a prospective pilot study. Clin Med: Oncol 2007, 1:73-80.
30. Eliaz I, Hotchkiss A, Fishman M. Rode D: The effect of modified citrus pectin on urinary excretion of toxic elements. Phytother Res 2006, 20:859-864.
31. Zhao ZY, Liang L, Fan X, Yu Z, Hotchkiss AT, Wilk BJ, Eliaz I: The role of modified citrus pectin as an effective chelator of lead in children hospitalized with toxic lead levels. Altern Ther Health Med 2008, 14:34-38.
32. Liu L, Chahroudi A, Silvestri G, Wernett ME, Kaiser WJ, Safrit JT, Komoriya A, Altman JD, Packard BZ, Feinberg MB: Visualization and quantification of T cell-mediated cytotoxicity using cell-permeable fluorogenic caspase molecules. Nat Med 2000, 8:185-189.
33. Jerome KR, Sloan DD, Aubert M: Measuring T-cell mediated cytotoxicity using antibody to activated caspases. Nat Med 2002, 9:4-5.
34. Nair PK, Rodriguez S, Ramachandran R, Alamo A, Melnick SJ, Escalon E, Garcia PI Jr, Wnuk SF, Ramachandran C: Immune stimulating properties of a novel polysaccharide from the medicinal plant Tinospora cordifolia. Int. Immunopharmacol 2004, 4:1645-59.
35. Inngjerdingen KT, Debes SC, Inngjerdingen M, Hokputsa S, Harding SE, Rolstad B, Michaelsen TE, Diallo D, Paulsen BS: Bioactive pectic polysaccharides from Glinus oppositifolius (L.) Aug. DC., a Malian medicinal plant, isolation and partial characterization. J Ethnopharmacol 2005, 101(1-3):204-14.
36. Inngjerdingen KT, Patel TR, Chen X, Kenne L, Allen S, Morris GA, Harding SE, Matsumoto T, Diallo D, Yamada H, Michaelsen TE, Inngjerdingen M, Paulsen BS: Immunological and structural properties of a pectic polymer from Glinus oppositifolius. Glycobiology 2007, 17(12): 1299-310.
37. Hotchkiss AT, Hicks KB: Analysis of pectate lyase-generated oligogalacturonic acids by high-performance anion-exchange chromatography and pulsed amperometric detection. Carbohydr Res 2003, 247:1-7.
38. Robertson MJ, Ritz J: Biology and clinical relevance of natural killer cells. Blood 1990, 76(12):2421-2438.
39. Takeda K, Okumura K. CAM and NK Cells. eCAM, 2010, 1(1):17-27.

The various embodiments will be described in detail with reference to the accompanying drawings. Wherever possible, the same reference numbers will be used throughout the drawings to refer to the same or like parts.

It is advantageous to define several terms before describing the invention. It should be appreciated that the following definitions are used throughout this application.

While the present invention has been disclosed both generically, and with reference to specific alternatives, those alternatives are not intended to be limiting unless reflected in the claims set forth below. The invention is limited only by the provisions of the claims, and their equivalents, as would be recognized by one of skill in the art to which this application is directed.

### ASPECTS OF THE INVENTION

1. A method of inducing an increase in activated T-cytotoxic/suppressor cells in a mammal in need of same comprising;
   selecting a mammal in need of an increase in activated T-cytotoxic/suppressor cells by identifying said mammal as one having lower than average activated T-cytotoxic/ suppressor cell titer for a representative population of said mammal,
   and administering to said mammal an amount of modified citrus pectin (MCP) effective to increase the activated T-cytotoxic/suppressor cell titer in said mammal.
2. A method of inducing an increase in activated B-cells in a mammal in need of same comprising;
   selecting a mammal in need of an increase in activated B-cells by identifying said mammal as one having lower than average B- cell titer for a representative population of said mammal,
   and administering to said mammal an amount of MCP effective to increase the activated B-cell titer in said mammal.
3. A method of inducing an increase in activated NK-cells cells in a mammal in need of same comprising;
   selecting a mammal in need of an increase in activated NK-cells by identifying said mammal as one having lower than average activated NK-cell titer for a representative population of said mammal,
   and administering to said mammal an amount of modified citrus pectin (MCP) effective to increase the activated NK-cell titer in said mammal.
4. The method of Aspect 3, wherein said NK cells activated by the administration of MCP to said mammal exhibit the ability to induce cell death in cancer cells.

## Claims

1. Modified citrus pectin (MCP) for use in a method of inducing an increase in activated NK-cells in a mammal in need of same comprising:
selecting a mammal in need of an increase in activated NK-cells by identifying said mammal as one having lower than average activated NK-cell titer for a representative population of said mammal;
and administering to said mammal an amount of MCP effective to increase the activated NK-cell titer in said mammal.

2. MCP for the use of claim 1, wherein said NK-cells activated by the administration of MCP to said mammal exhibit the ability to induce cell death in cancer cells.

3. MCP for the use of claim 2 in a method of treating cancer.

4. MCP for the use of claim 3 in a method of treating leukemia.

5. MCP for the use of claim 4, wherein the leukemia is myeloid leukemia.
